# EUROPEAN PATENT APPLICATION

(11) **EP 2 404 582 A1**
(43) Date of publication of application: **11.01.2012**
(21) Application number: 10748615.1
(22) Date of filing: 15.02.2010
(51) Int. Cl.: A61F 13/15, A61F 13/49

(54) **DISPOSABLE DIAPER**

(30) Priority: 03.03.2009 JP 2009049914
(71) Applicant: Unicharm Corporation, Ehime 799-0111 (JP)
(72) Inventor: YAMASHITA, Mariko, Kanonji-shi Kagawa 769-1602 (JP); BABA, Toshimitsu, Kanonji-shi Kagawa 769-1602 (JP)
(74) Representative: Peter, Julian
(86) International application number: PCT/JP2010/052221
(87) International publication number: WO 2010/101019

(57) **Abstract**

The present invention provides a disposable diaper free from a possibility that the diaper might be significantly displaced and assuring the improved breathability. At least one of front and rear waist regions 13, 14 is elastic and this waist region 13 is divided into a first elastic zone 13A extending in a transverse direction X along a waist-opening's periphery and a second elastic zone 13B defined between the first elastic zone 13A and an inner end of the waist region 13 and extending in the transverse direction X. The second elastic zone 13B is provided with waist elastics 24 and consequently the second elastic zone 13B has a tensile stress higher than that of the first elastic zone 13A. The waist region 13 is formed with a plurality of breathing pathways 25 extending in a longitudinal direction Y and the breathing pathways 25 in the second elastic zone 13B respectively have a width dimension smaller than that in the first elastic zone 13A.

## Description

### {Technical Field}

The present invention relates to absorbent wearing articles and, more particularly, to disposable diapers assuring an appropriate fit to the wearer's body by front and rear waist regions having elastic stretch properties and simultaneously assuring a high breathability.

### {Background}

Disposable diapers being composed so as to satisfy both requirements for breathability and fit are generally known. For example, PTL 1 discloses a disposable diaper wherein at least one of front and rear waist regions is provided on its skin-facing side with a breathable sheet bonded thereto.

### {Citation List}

### {Patent Literature}

{PTL 1} JP 2000-189454 A

### {Summary}

### {Technical Problem}

According to the invention disclosed in PTL 1, a hydrophilic breathable sheet having a plurality of openings is permanently bonded to the skin-facing side of at least one of the front and rear waist regions to improve the breathability. Overlapping the breathable sheet, at least one of front and rear peripheries of a waist-opening is provided with a plurality of strand-like waist elastics extending in a transverse direction to prevent at least the one of the waist regions from being displaced.

However, due to such an arrangement of the strand-like waist elastics and the breathable sheet in an overlapping fashion, wrinkles of various sizes should be formed on the skin-facing side of the breathable sheet and the breathable sheet should be partially spaced from the wearer's body, and eventually bodily fluids might leak out through these void spaces.

If none of the strand-like waist elastics is provided in order to avoid the above-mentioned problem, a fit of the front and rear waist regions to the wearer' s skin should be deteriorated and the diaper should be displaced particularly due to a movement of the wearer's thighs during use thereof, and eventually bodily fluids might leak sideways.

An object of this invention is to provide a disposable diaper free from a possibility that the diaper might be significantly displaced and assuring an improvedbreathability.

### {Solution to Problem}

According to this invention, there is provided a disposable diaper having a longitudinal direction and a transverse direction, and including a skin-facing side, a garment-facing side, a front waist region, a rear waist region, a crotch region extending between the front and rear waist regions, a waist-opening and a pair of leg-openings wherein at least one of the front and rear waist regions is elastically stretchable.

The present invention is **characterized in that**: the one of the front and rear waist regions includes an inelastic sheet lying on the garment-facing side and a moisture-pervious elastic sheet lying on the skin-facing side; a plurality of breathing pathways extending in the longitudinal direction are formed between the inelastic sheet and the elastic sheet; the one of the front and rear waist regions is divided into a first elastic zone extending in the transverse direction along a periphery of the waist-opening and a second elastic zone defined between the first elastic zone and an inner end of the one of the front and rear waist regions so as to extend in the transverse direction and provided with waist elastics extending in the transverse direction so as to have a tensile stress higher than that of the first elastic zone; and the breathing pathways in the second elastic zone respectively have a width dimension smaller than that of the respective breathing pathways in the first elastic zone.

This invention includes, in addition to the above-mentioned characterizing embodiment, preferred embodiments as follows:
(1) The inelastic sheet and the elastic sheet are bonded to each other with a plurality of adhesive lines extending substantially in parallel to one another in the longitudinal direction and each of the breathing pathways is defined by a tubular shape formed by making ridges of segments of the inelastic sheet being present between each pair of the adjacent adhesive lines.
(2) The diaper further includes an annular waist panel defining the front and rear waist regions and an absorbent chassis attached to the skin-facing side of the annular waist panel so as to extend across the crotch region into the front and rear waist regions and containing therein a liquid-absorbent core.
(3) The waist elastics include upper waist elastics lying above one of front and rear ends of the absorbent chassis so as to extend continuously between opposite side edges of the one of the front and rear waist regions and a pair of lower waist elastics spaced from and opposed to each other in the transverse direction and these lower waist elastics extend from the opposite side edges of the one of the front and rear waist regions to the outside of the opposite side edges of the liquid-absorbent core.
(4) The upper waist elastics have a tensile stress different from that of the lower waist elastics.
(5) The one of the front and rear waist regions is formed with a plurality of breathing apertures extending through the elastic sheet and the inelastic sheet.
(6) The waist elastic includes a plurality of strand-like elastic members extending in the transverse direction.
(7) The elastic sheet has a hydrophilic nature.

### {Advantageous Effects of Invention}

According to this invention, the elastic sheet lies on the skin-facing side of at least one of the front and rear waist regions so that the front and rear waist regions as a whole may have an appropriate fit and these waist regions are formed with the breathing pathways adapted to breathe out vapor generated within the diaper, assuringhighbreathability. Thiswaistregion is divided into the first elastic zone and the second elastic zone provided with the waist elastics so that the second elastic zone may have the tensile stress correspondingly higher than that of the first elastic zone and the second elastic zone should not be significantly displaced even by a movement of the wearer' s thighs.

### {Brief Description of Drawings}

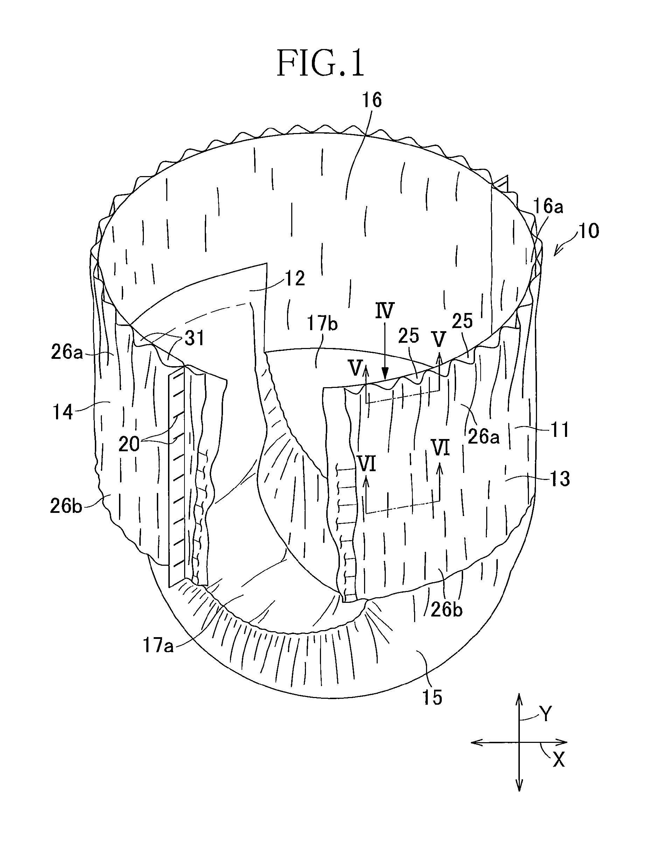

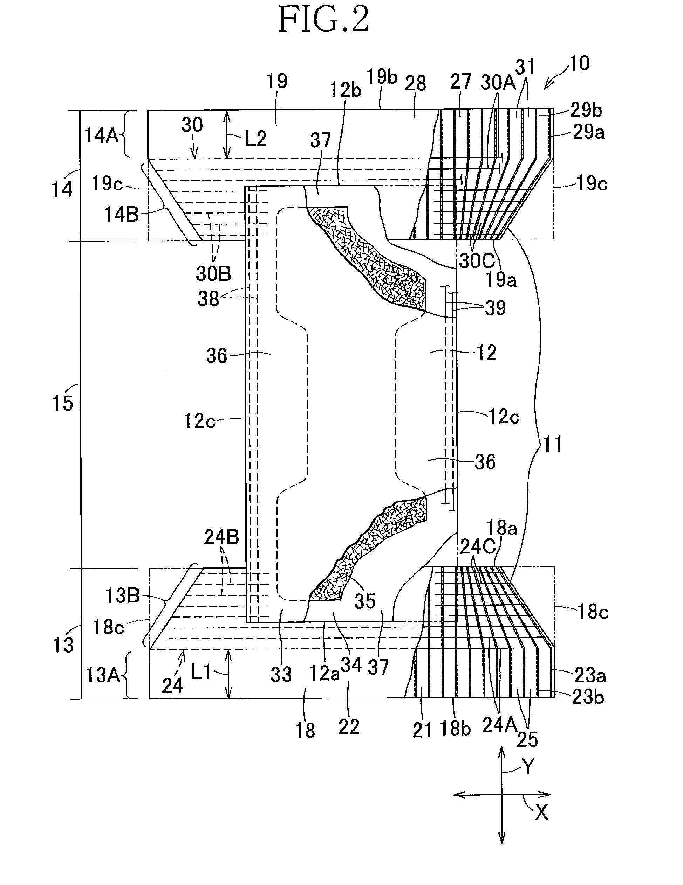

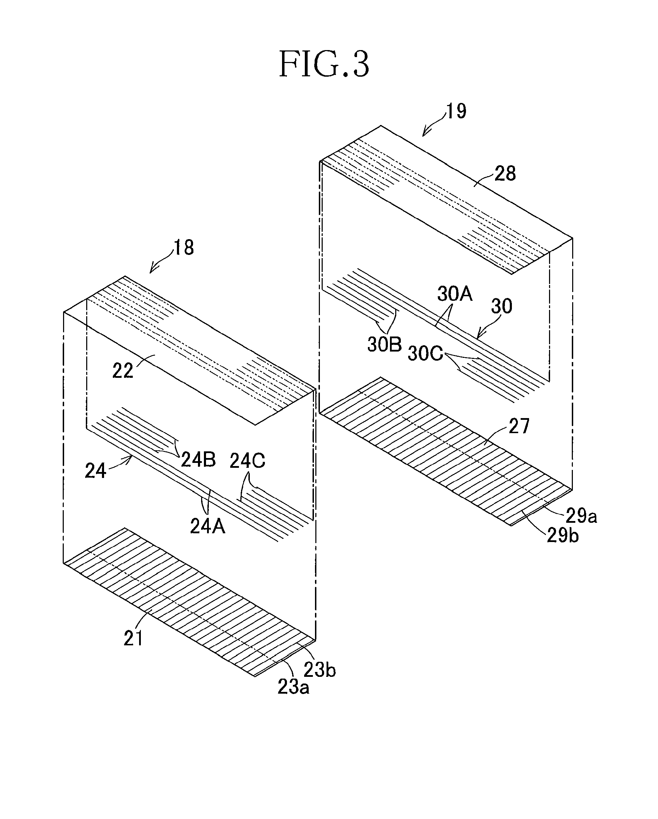

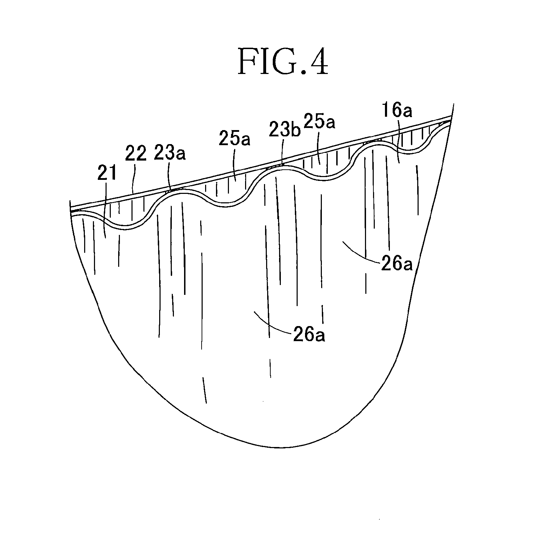

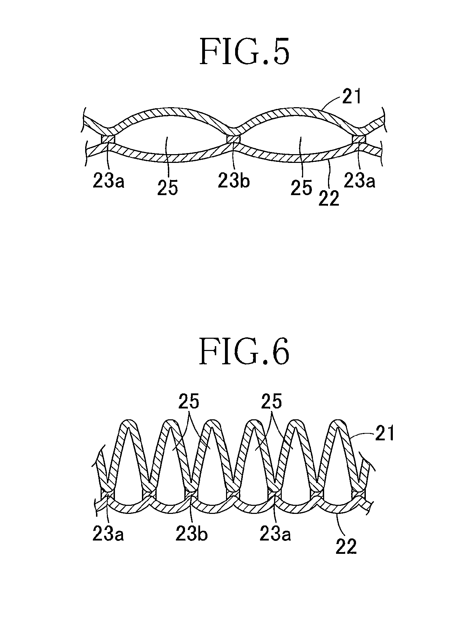

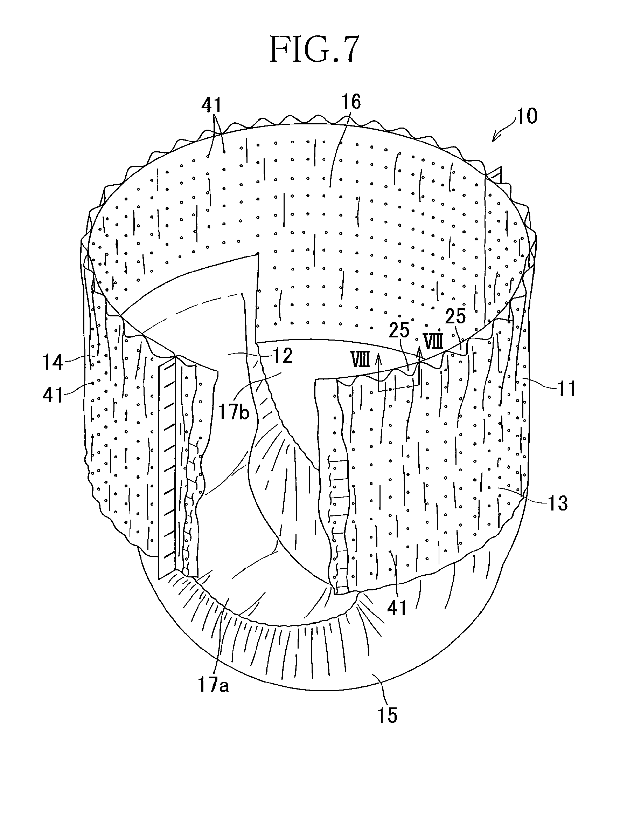

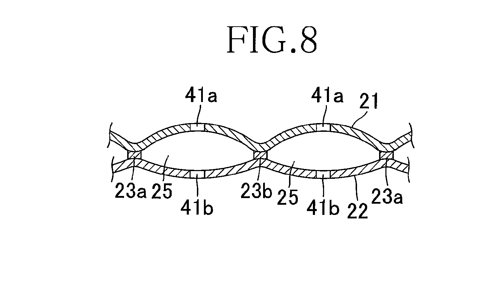

{Fig. 1} A partially cutaway perspective view of a diaper according to a first embodiment.
{Fig. 2} A developed plan view of the diaper.
{Fig. 3} An exploded perspective view of a front waist panel.
{Fig. 4} A scale-enlarged view as viewed in an arrow direction IV indicated in Fig. 1.
{Fig. 5} A sectional view taken along line V-V in Fig. 1.
{Fig. 6} A sectional view taken along line VI-VI in Fig. 1.
{Fig. 7} A partially cutaway perspective view of a diaper according to a second embodiment.
{Fig. 8} A sectional view taken along line VIII-VIII in Fig. 7.

### {Description of Embodiments}

### <First Embodiment>

Fig. 1 is a partially cutaway perspective view of a diaper 10 according to a first embodiment as viewed from a lateral side, Fig. 2 is a developed plan view of the diaper 10 developed in a longitudinal direction Y and a transverse direction X with side seams broken and Fig. 3 is an exploded perspective view of a front waist panel. In the diaper shown in Fig. 1, first elastic zones 13A, 14A of front and rear waist regions 13, 14, respectively, are illustrated in a stretched state in the transverse direction X but second elastic regions 13B, 14B are illustrated in a contracted state. Respective side edges 18c, 18c, 19c, 19c thereof are indicated by imaginary lines.

Referring to Fig. 1, the diaper 10 has the longitudinal direction and the transverse direction X, and includes a skin-facing side, a garment-facing side a annular waist panel 11 adapted to be annulus-shaped with the diaper 10 put on the wearer's body, an absorbent chassis 12 attached to an inner surface of the annular waist panel 11, i.e. , to the skin-facing side of the diaper 10, a front waist region 13, a rear waist region 14, a crotch region 15 extending between the front and rear waist regions 13, 14, a waist-opening 16 and a pair of leg-openings 17a, 17b.

As will be apparent from Figs. 1 and 2, the annular waist panel 11 is composed of a front waist panel 18 defining the front waist region 13 and a rear waist panel 19 defining the rear waist region 14.

The front waist panel 18 is contoured by an inner end 18a extending in the transverse direction, an outer end 18b opposed to the inner end 18a in the longitudinal direction Y and both side edges (both side edges of the front waist region 13) 18c, 18c extending in the longitudinal direction Y between the inner end 18a and the outer end 18b to take on a transversely long rectangle. In a similar way, the rear waist panel 19 is contoured by an inner end 19a extending in the transverse direction, an outer end 19b opposed to the inner end 19a in the longitudinal direction Y and both side edges (both side edges of the rear waist region 14) 19c, 19c extending in the longitudinal direction Y between the inner end 19a and the outer end 19b to taken on a transversely long rectangle. The respective opposite side edges 18c, 19c of the front and rear waist panels 18, 19 are illustrated to be in a contracted state under a contractile effect of front and rear waist elastics 24, 30 to be hereinafter described. Shapes of the front and rear waist panels 18, 19 before contraction are indicated by imaginary lines.

The opposite side edges 18c, 18c of the front waist panel 18 and the opposite side edges 19c, 19c of the rear waist panel 19 are put flat and joined together along side seams defined by a plurality of joining spots arranging intermittently in the longitudinal direction Y, joining spots being formed by known art, for example, various types of heat sealing techniques inclusive of heat embossing, ultrasonic sealing and the like.

Referring to Figs. 2 and 3, the front waist panel 18 includes a first inelastic sheet 21 lying on the garment-facing side and a first elastic sheet 22 lying on the skin-facing side and having a moisture-pervious property.

The first elastic sheet 22 is bonded to the first inelastic sheet 21 with a plurality of adhesive lines 23a, 23b formed of hot melt adhesives applied to the inner surface of the first inelastic sheet 21. The adhesive lines 23a, 23b are of a stripe pattern and each pair of the adjacent adhesive lines 23a, 23b are spaced from each other by a given dimension in the transverse direction X.

The front waist region 13 is divided into a first elastic zone 13A extending along a waist-opening periphery 16a in the transverse direction X and a second elastic zone 13B defined between the first elastic zone 13A and a lower end of the front waist region 13 (corresponding to the inner end 18a of the front waist panel 18) and extending in the transverse direction X.

Front waist elastics 24 including a plurality of strand-like elastic members extending in the transverse direction X is sandwiched between the first inelastic sheet 21 and the first elastic sheet 22 in the second elastic zone 13B. The first elastic sheet 22 lying on the skin-facing side assures the front waist region 13 as a whole to fit the wearer's body appropriately. The second elastic zone 13B provided with the front waist elastics 24 has a tensile stress sufficiently higher than a tensile stress of the first elastic zone 13A so that the second elastic zone 13B should not be significantly displaced even by the movement of the wearer's thighs. In contrast, the first elastic zone 13A is not provided with the waist elastics 24 and therefore not influenced by the contraction of the elastics 24. Consequently, the first elastic sheet 22 should not get wrinkledso as to space the first elastic sheet 22 from the wearer's skin, and bodily fluids should not leak out from the diaper.

The front waist elastics 24 are composed of an upper waist elastics 24A extending in the transverse direction X along a front end 12a of the absorbent chassis 12 and a pair of lower waist elastics 24B, 24C both underlying the upper waist elastics 24A and spaced from each other in the transverse direction X.

The first inelastic sheet 21 is formed between each pair of the adjacent adhesive lines 23a, 23b with ridges 26a, 26b which are continuous to each other in the longitudinal direction Y (See Fig. 1) and these continuous ridges 26a, 26b cooperate with the first elastic sheet 22 to define a pathway 25 extending in the longitudinal direction Y. The waist-opening's periphery 16a is formed with upper openings of the respective pathways 25.

Referring to Fig. 2, the second elastic zone 13B is contracted under the tensile stress of the front waist elastics 24 and, inconsequence, a width dimension of the breathing pathway 25 being smaller in the second elastic zone 13B than in the first elastic zone 13A and the ridges 26b in the second elastic zone 13B is smaller than the ridges 26a.

The rear waist panel 19 includes a second inelastic sheet 27 lying on the garment-facing side and a second elastic sheet 28 lying on the skin-facing side. The second elastic sheet 28 is bonded to the second inelastic sheet 27 with adhesive lines 29a, 29b formed of hot melt adhesives applied to the inner surface of the second inelastic sheet 27. The adhesive lines 29a, 29b are provided in the form of independent stripe lines extending in parallel to one another in the longitudinal direction Y wherein each pair of the adjacent adhesive lines 29a, 29b are spaced from each other by a predetermined dimension in the transverse direction.

The rear waist region 14 is divided into a first elastic zone 14A extending along a waist-opening periphery 16a in the transverse direction X and a second elastic zone 14B defined between the first elastic zone 14A and a lower end of the rear waist region 14 (corresponding to the inner end 19a of the rear waist panel 19) and extending in the transverse direction X.

Rear waist elastics 30 including a plurality of strand-like elastic members extending in the transverse direction X are sandwiched between the second inelastic sheet 27 and the first elastic sheet 28 in the second elastic zone 14B. As in the front waist region 13, the second elastic sheet 28 lying on the skin-facing side assures the rear waist region 14 as a whole to fit the wearer's body appropriately. The second elastic zone 14B provided with the rear waist elastics 30 has a tensile stress sufficiently higher than a tensile stress of the first elastic zone 14A so that the second elastic zone 14B should not be significantly displaced even by the movement of the wearer's thighs. In contrast, the first elastic zone 14A is not provided with the rear waist elastics 30 and, therefore, the second elastic sheet 28 should not be influenced by the contraction of the rear waist elastics 30. Consequently, the second elastic sheet 28 should not get wrinkled so as to space the first elastic sheet 28 from the wearer's skin, and bodily fluids should not leak out from the diaper.

The rear waist elastics 30 are composed of an upper waist elastics 30A extending in the transverse direction X along a rear end 12b of the absorbent chassis 12 and a pair of lower waist elastics 30B, 30C both underlying the upper waist elastics 30A and opposed to and spaced from each other in the transverse direction X.

The second inelastic sheet 27 is formed between each pair of the adjacent adhesive lines 29a, 29b with ridges 26a, 26b which are continuous to each other in the longitudinal direction Y and these continuous ridges 26a, 26b cooperate with the second elastic sheet 28 to define a tubular breathing pathway 31 extending-through in the longitudinal direction Y. The waist-opening's periphery 16a is formed with upper openings of the respective breathing pathways 31.

The second elastic zone 14B is contracted under the tensile stress of the waist elastics 30 and, in consequence, a width dimension of the breathing pathway 31 is smaller in the second elastic zone 14B than in the first elastic zone 14A. The ridge 26b in the second elastic zone 14B is smaller than the ridge 26a in the first elastic zone 14A.

In order that the respective adhesive lines 23a, 23b, 29a, 29b of the front and rear waist panels 18, 19 can be prevented from coming in contact with the wearer' s skin, it is preferable that the waist-opening's periphery 16a should not be coated with these adhesive lines. In the front and rear waist panels 18, 19, respective spacing dimensions L1, L2 measured from the waist-opening' s periphery to the outermost regions of the upper waist elastics 24A, 30A, respectively, are preferably in a range of 30 to 50mm. This is because, with the spacing dimensions L1, L2 of 30mm or less, the tensile stress of the front and rear waist elastics 22, 28 may act upon the first and second elastic sheets 22, 28 in the vicinity of the waist-opening's periphery 16a.

In the front and rear waist panels 18, 19, a width dimension of the first elastic sheet 22 in the transverse direction X is smaller than that of the first inelastic sheet 21 and a width dimension of the second elastic sheet 28 in the transverse direction X is smaller than that of the second inelastic sheet 27. With such a dimensional relationship, the first and second elastic sheets 22, 28 may be bonded to the first and second inelastic sheets 21, 27 after the first and second elastic sheets 22, 28 have been stretched to the width dimension of the first and second inelastic sheets 21, 27, respectively, with the adhesive lines 23a, 23b, 29a, 29b to assure that the first and second inelastic sheets 21, 27 get wrinkled and, in consequence, the ridges continuously extending in the longitudinal direction Y as has previously been described.

Referring to Figs. 2 and 3, the lower waist elastics 24B, 24C, 30B, 30C in the front and rear waist panels 18, 19 extend from the side edges of the respective waist panels 18, 19 to the opposite side edges of a liquid-absorbent core 35 without extending across the liquid-absorbent core 35. Even when the lower waist elastics 24B, 24C, 30B, 30C are in a contracted state, since the lower waist elastics 24B, 24C, 30B, 30C are not extending across the liquid-absorbent core 35, the tensile stress thereof should not act on the liquid-absorbent core 35 to get wrinkles thereon and the absorbing capacity of the liquid-absorbent core 35 should not be deteriorated.

It is possible to set the tensile stress of the upper waist elastics 24A, 30A to be higher than the tensile stress of the lower waist elastics 24B, 24C, 30B, 30C for the purpose of preventing the absorbent chassis 12 from being displaced in the front-back direction and it is also possible to set the tensile stress of the lower waist elastics 24B, 24C, 30B, 30C to be higher than that of the upper waist elastics 24A, 30A for the purpose of preventing the absorbent chassis 12 from being displaced in the transverse direction X.

While the strand-like elastic members are used as the front and rear waist elastics 24, 30 in the present embodiment, it is possible to use sheet-like elastic members so far as these sheet-like elastic members have the same tensile stress as that of the strand-like elastic members.

The first and second elastic sheets 22, 28 may be formed of a laminated fibrous nonwoven fabric being moisture-pervious and elastically stretchable and preferably having a hydrophilic nature, and the first and second inelastic sheets 21, 27 may be formed, for example, of a laminate sheet composed of a hydrophobic fibrous nonwoven fabric and a moisture-pervious or moisture-impervious plastic sheet. When the first and second elastic sheets 22, 28 have a hydrophilic nature, sweats will be quickly absorbed by these sheets and thereby a sweat-absorbing function of the diaper will be improved.

More specifically, the first and second elastic sheets 22, 28 are formed preferably by mechanically stretching a mixed fibrous nonwoven fabric composed of elastomer fibers and inelastic thermoplastic fibers on a stretching means of known art such as a stretching roll and bonding these elastic sheets 22, 28 under tension in the transverse direction X at a stretch ratio of about 1.5 to 3.0 fold to the first and second inelastic sheets 21, 27, respectively. The elastomer fibers used for the first and second elastic sheets 22, 28 are urethane- or polyethylene-based and present in the mixed fibrous nonwoven fabric as a whole preferably at a mixing ratio in a range of 20 to 75% by mass. This is because if the elastomer fibers are mixed at a mixing ratio of 20% by mass or less, the ratio of the thermoplastic fibers will exceed a permissible limit and, in consequence, the elastic nonwoven fabric will be apt to strain and if the elastomer fibers are mixed at a mixing ratio of 75% by mass or more, the sheets as a whole will be apt to cling to the wearer's skin and a feel of the sheets against the wearer's skin will be deteriorated.

The absorbent chassis 12 is contoured by the front end 12a extending in the transverse direction X, the rear end 12b opposed to the front end 12a in the longitudinal direction Y and opposite side edges 12c, 12c extending in the longitudinal direction Y between the front and rear ends 12a, 12b so as to define a longitudinally long rectangle.

The absorbent chassis 12 includes a liquid-pervious inner sheet 33 lying on the skin-facing side, a liquid-impervious outer sheet 34 lying on the garment-facing side and the liquid-absorbent core 35 sandwiched between the inner sheet 33 and the outer sheet 34 and composed of fluff wood pulp and superabsorbent polymer (SAP) mixed together. The liquid-absorbent core 35 is previously molded in a desired shape and, if desired, the entirety thereof may be wrapped with a liquid-dispersant sheet (not shown).

The inner sheet 33 and the outer sheet 34 extend outward beyond the peripheral edge of the liquid-absorbent core 35 and are permanently bonded to each other with hot melt adhesives so as to form side flaps 36 extending in the longitudinal direction Y outside the respective side edges of the liquid-absorbent core 35 and end flaps 37 extending in the transverse direction X outside the front and rear ends of the liquid-absorbent core 35. The end flaps 37 are bonded to the respective inner surfaces of the front and rear waist panels 18, 19 with hot melt adhesives. When the diaper 10 is put on the wearer's body, the absorbent chassis 12 is in a state of being suspended from the annular waist panel 11.

The respective side flaps 36 are provided along outer side edges thereof i.e., along the opposite side edges 12c of the absorbent chassis 12 with strand-like elastics 38, 39 extending in the longitudinal direction Y. Though not shown, it is possible to provide the side flaps 36 with barrier cuffs serving to prevent bodily fluids from leaking sideways wherein the barrier cuffs may be formed of a separately prepared liquid-impervious sheet member or by extending the outer sheet 34 beyond the inner sheet 33 in the transverse direction X and folding back these extended portions.

While both the front and rear waist regions 13, 14 are composed in the aforementioned manner to form the breathing pathways 25, 31 according to this embodiment, it is possible to compose at least one of the front and rear waist regions 13, 14 in this manner. While the front and rear waist regions 13, 14 are respectively defined by the front and rear waist panels 18, 19 which are separately prepared according to this embodiment, it is possible to form these waist panels 18, 19 of a single continuous sheet member so that this single sheet member may define the integral outer surface of the diaper 10.

Fig. 4 is a partially scale-enlarged view as viewed in an arrow direction IV indicated in Fig. 1, Fig. 5 is a sectional view taken along line V-V in Fig. 1 and Fig. 6 is a sectional view taken along line VI-VI in Fig. 1. While Figs. 4 through 6 illustrate the arrangement of the front waist region 13 only, it should be appreciated that the rear waist region 14 is also arranged in the similar manner.

Referring to Figs. 4 and 5, the breathing pathways 25 extend across the front waist region 13 in the longitudinal direction Y, and vapor generated within the diaper 10 is let off outward from the upper openings and the lower openings (not shown). In this way, substantially no vapor stays within the diaper 10 and a high breathability is assured. Considering that the wearer is apt to sweat in the vicinity of the waist-opening' s periphery, sweats once absorbed by the diaper can be effectively let out in the form of vapor.

### <Second Embodiment>

Fig. 7 is a perspective view similar to Fig. 1, showing a second embodiment of this invention and Fig. 8 is a sectional view taken along line VIII-VIII in Fig. 7.

According to this embodiment, the front and rear waist regions 13, 14 are formed substantially over the entire areas thereof with a plurality of breathing apertures 41. Specifically, the breathing apertures 41 extend through the first and second inelastic sheets 21, 27 as well as the first and second elastic sheets 22, 28 between each pair of the adjacent adhesive lines 23a, 23b, 29a, 29b wherein the ridges 26a, 26b of the first and second inelastic sheets 21, 27 are formed with outer breathing apertures 41a and regions of the first and second elastic sheets 22, 28 opposed to the ridges 26a, 26b are formed with inner breathing apertures 41b.

According to this embodiment, the vapor generated within the diaper 10 flows via the respective inner breathing apertures 41b into the breathing pathways 25 and is let out from the diaper 10 from the upper openings 25a and the lower openings of the breathing pathways 25 or directly from the outer breathing apertures 41a. In this way, the breathability can be further improved and stuffiness which otherwise would be generated within the diaper 10 can be prevented.

In order to prevent bodily fluids from leaking out through the breathing apertures 41, a width dimension of the respective outer breathing apertures 41 is preferably larger than a width dimension of the respective inner side breathing apertures 41b, in other words, the respective breathing apertures 41 are preferably tapered from the outer side to the inner side. With this arrangement, the vapor generated within the diaper 10 can be smoothly let out through the inner side breathing apertures 41b to improve the breathability and leakage of bodily fluids through the outer side breathing apertures 41a can be effectively prevented.

To achieve such effect, the width dimension of the respective inner side breathing apertures 41b may be set preferably to a range of 0.01 to 0.26cm. To further improve the preventive effect against leakage of bodily fluids, it is possible to modify the arrangement of the breathing apertures so that the breathing apertures 41 extend not through the first and second inelastic sheets 21, 27 but through only the first and second elastic sheets 22, 28 and the inner side breathing apertures 41b intercommunicate with the breathing pathways 25.

While a plurality of adhesive lines 23a, 23b, 29a, 19b formed of hot melt adhesives and extending in the longitudinal direction define a plurality of tubular breathing pathways 25, 31 continuously extending in the longitudinal direction Y, respectively, according to the illustrated embodiments of this invention, it is possible to apply hot melt adhesives intermittently in the longitudinal direction Y so that each pair of the adjacent breathing pathways 25, 31 may intercommunicate with each other or to apply hot melt adhesives in zigzag-pattern so that the breathing pathways 25, 31 as a whole may intercommunicate with one another.

For the respective component members of the diaper 10 such as the elastic waist panel 11 and the absorbent chassis 12, various kinds of known material other than those described in this description may be selectively used. The diaper 10 is not limited to the pants-type disposable diaper but this invention is applicable also to a so-called open-type disposable diaper in which the front and rear waist regions 13, 14 are not previously joined together at the side seams.

### {Reference Signs List}

- 10: disposable diaper
- 11: annular waist panel
- 12: absorbent chassis
- 13: front waist region
- 14: rear waist region
- 15: crotch region
- 16: waist-opening
- 16a: waist-opening's periphery
- 17a, 17b: leg-openings' peripheries
- 18: front waist panel
- 18a: inner end of front waist panel (i.e., lower end of front waist region)
- 18c, 18c: opposite side edges of front waist panel (i.e., opposite side edges of front waist region)
- 19: rear waist panel
- 21, 27: inelastic sheets
- 22, 28: elastic sheets
- 23a, 23b, 23c, 29a, 29b, 29c: adhesive lines
- 24A, 30A: upper waist elastics
- 24B, 24C, 30B, 30C: lower waist elastics
- 25, 31: breathing pathways
- 35: liquid-absorbent core
- 41: breathing apertures

## Claims

1. A disposable diaper having a longitudinal direction and a transverse direction, and comprising a skin-facing side, a garment-facing side, a front waist region, a rear waist region, a crotch region extending between the front and rear waist regions, a waist-opening and a pair of leg-openings wherein at least one of the front and rear waist regions is elastic, the disposable diaper being **characterized in that**:
the one of the front and rear waist regions includes an inelastic sheet lying on the garment-facing side and a moisture-pervious elastic sheet lying on the skin-facing side;
a plurality of breathing pathways extending in the longitudinal direction are formed between the inelastic sheet and the elastic sheet;
the one of the front and rear waist regions is divided into a first elastic zone extending in the transverse direction along a periphery of the waist-opening and a second elastic zone defined between the first elastic zone and an inner end of the one of the front and rear waist regions so as to extend in the transverse direction and provided with waist elastics extending in the transverse direction so as to have a tensile stress higher than that of the first elastic zone; and
the breathing pathways in the second elastic zone respectively have a width dimension smaller than that of the respective breathing pathways in the first elastic zone.

2. The diaper defined by claim 1, wherein the inelastic sheet and the elastic sheet are bonded to each other with a plurality of adhesive lines extending substantially in parallel to one another in the longitudinal direction and each of the breathing pathways is defined by a tubular shape formed by making ridges of segments of the inelastic sheet being present between each pair of the adjacent adhesive lines.

3. The diaper defined by claim 1 or 2, further including an annular waist panel defining the front and rear waist regions and an absorbent chassis attached to the skin-facing side of the annular waist panel so as to extend across the crotch region into the front and rear waist regions and containing therein a liquid-absorbent core.

4. The diaper defined by any one of claims 1 through 3, wherein the waist elastics comprise upper waist elastics lying above the front or rear end of the absorbent chassis so as to extend continuously between opposite side edges of the one of the front and rear waist regions and a pair of lower waist elastics spaced from and opposed to each other in the transverse direction and these lower waist elastics extend from the opposite side edges of the one of the front and rear waist regions to the opposite side edges of the liquid-absorbent core.

5. The diaper defined by claim 4, wherein the upper waist elastics have a tensile stress different from that of the lower waist elastics.

6. The diaper defined by any one of claims 1 through 5, wherein the one of the front and rear waist regions is formed with a plurality of breathing apertures extending through the elastic sheet and the inelastic sheet.

7. The diaper defined by any one of claims 1 through 6, wherein the waist elastic comprises a plurality of strand-like elastic members extending in the transverse direction.

8. The diaper defined by any one of claims 1 through 7, wherein the elastic sheet has a hydrophilic nature.
